# EUROPÄISCHE PATENTSCHRIFT

(11) **EP 3 510 013 B1**
(45) Veröffentlichungstag und Bekanntmachung des Hinweises auf die Patenterteilung: **11.11.2020**
(21) Anmeldenummer: 17758559.3
(22) Anmeldetag: 01.09.2017
(51) Int. Cl.: C07C 213/02, C07C 209/48

(54) **VERFAHREN ZUR NITRILHYDRIERUNG IN GEGENWART EINES AUF ZRO2 GETRÄGERTEN RUTHENIUM-KATALYSATORS**
METHOD FOR HYDRATING NITRILES IN THE PRESENCE OF A RUTHENIUM CATALYST CARRIED ON ZRO2
PROCÉDÉ D'HYDROGÉNATION DE NITRILES EN PRÉSENCE D'UN CATALYSEUR AU RUTHÉNIUM SUPPORTÉ SUR ZRO2

(30) Priorität: 08.09.2016 EP 16187732
(43) Veröffentlichungstag der Anmeldung: 17.07.2019
(73) Patentinhaber: BASF SE, 67056 Ludwigshafen am Rhein (DE)
(72) Erfinder: EIDAMSHAUS, Christian, 67056 Ludwigshafen (DE); KRUG, Thomas, 67056 Ludwigshafen (DE); MELDER, Johann-Peter, 67056 Ludwigshafen (DE); PASTRE, Joerg, 67056 Ludwigshafen (DE); BEBENSEE, Regine Helga, 67056 Ludwigshafen (DE); JAEGLI, Stephanie, 67056 Ludwigshafen (DE)
(74) Vertreter: BASF IP Association
(86) Internationale Anmeldenummer: PCT/EP2017/071936
(87) Internationale Veröffentlichungsnummer: WO 2018/046393

(56) Entgegenhaltungen:
- EP-A1- 2 684 862
- DE-A1- 10 216 745
- GUANGYIN FAN ET AL: "Highly Efficient Hydrogenation of Methyl Propionate to Propanol over Hydrous Zirconia Supported Ruthenium", CHINESE JOURNAL OF CHEMISTRY, Bd. 29, Nr. 2, 1. Februar 2011 (2011-02-01), Seiten 229-236, XP055121946, ISSN: 1001-604X, DOI: 10.1002/cjoc.201190071

## Beschreibung

Die vorliegende Erfindung betrifft ein Verfahren zur Hydrierung von Nitrilen mit Wasserstoff in Gegenwart eines auf ZrO₂-geträgerten Ruthenium-Katalysators.

Die Hydrierung von Nitrilen ist der wichtigste Prozess um primäre Amine herzustellen. Nitrilhydrierungen im industriellen Maßstab werden typischerweise an Cobalt-haltigen Festbettkatalysatoren oder in Gegenwart von Suspensionskatalysatoren wie Raney-Nickel und Raney-Cobalt durchgeführt, und sind aus der Literatur bekannt.

Nur wenige Beispiele beschreiben die Hydrierung von Nitrilen an Ruthenium-haltigen Katalysatoren.

C.Ortiz-Cervantes, I.lyanez, J.J. Garcia et al beschreiben in J. Phys. Org. Chem. 2013, 25, 902-907 den Einsatz von Ruthenium-Nanopartikel in der Hydrierung von Acetonitril, Benzonitril und Propionitril.

In WO96/23802 wird die Hydrierung in Gegenwart von homogen gelösten Ru-Katalysatoren mit Phosphan-Liganden beschrieben - zum Beispiel in der Hydrierung von Adipodintril.

Ein Beispiel für einen geträgerten Rutheniumkatalysator wird in EP-A 2684862 beschrieben. Hier wird die Batch-Hydrierung von Iminodiacetonitril zur Herstellung von Diethylentriamin (DE-TA) beschrieben. Hierbei wird ein auf Al₂O₃ geträgerter Rutheniumkatalysator eingesetzt. Ein auf ZrO₂ geträgerter Ruthenium Katalysator wird jedoch nicht beschrieben.

Bei der Hydrierung von Nitrilen werden häufig Co-Festbett-Katalysatoren, wie es beispielsweise in Ullmans's Encyclopedia of Industrial Chemistry "Amines, Aliphatic", DOI: 10.1002/14356007.a2_001 beschrieben ist, eingesetzt. Einige Nitrile neigen, unter den für Hydrierungen typischen Reaktionsbedingunen dazu Acrylnitril oder Blausäure freizusetzen. Solche Nitrile lassen sich typischerweise nur mit geringen Raum-Zeit-Ausbeuten hydrieren. Beispiele sind N,-N,-Biscyanethylalkylamine, cyanethylierte Alkohole und alpha-Aminonitrile.

Die Hydrierung solch anspruchsvoller Nitrile erfolgt in der Regel effizient nur an Suspensionskatalysatoren. Beispielsweise wird die Hydrierung von N,N-Biscyanethylmethylamin an Raney-Metall-Katalysatoren in EP0363843 und von Mikolajewska et al. Acta Pol. Pharm, 1966 beschrieben.

Effiziente Festbettkatalysatoren, die die genannten Nitrile in guten Raum-Zeit-Ausbeuten hydrieren sind nicht beschrieben.

Die Aufgabe der vorliegenden Erfindung ist es daher ein Festbettverfahren zur Hydrierung bereit zu stellen, das es nicht nur ermöglicht in guten Raum-Zeit-Ausbeuten Nitrile im Allgemeinen zu hydrieren, sondern die gleichzeitig auch anspruchsvolle Nitrile wie beispielsweise N,-N,-Biscyanethylalkylamine, cyanethylierte Alkohole und alpha-Aminonitrile mit gleichguten Raum-Zeit-Ausbeuten zu hydrieren wie einfache Nitrile. Des Weiteren soll der Katalysator in einem solchen Verfahren lange hohe Aktivität aufweisen und nur langsam deaktivieren.

Diese Aufgabe wird gelöst durch ein Verfahren zur Hydrierung von Nitrilen in Gegenwart von Wasserstoff und einem Ruthenium-Festbettkatalysator, der auf ZrO₂ geträgert ist.

Vorteilhaft ist das erfindungsgemäße Verfahren, wenn der eingesetzte Ru-Festbettkatalysator 0,05 bis 20 Gew-% Ruthenium bezogen auf das Gesamtgewicht des Katalysators enthält.

Vorteilhaft ist das erfindungsgemäße Verfahren, wenn das Nitrilhydrierverfahren kontinuierlich betrieben wird.

Vorteilhaft ist das erfindungsgemäße Verfahren, wenn die zu hydrierenden Nitrile Dinitrile sind.

Vorteilhaft ist das erfindungsgemäße Verfahren, wenn die zu hydrierenden Nitrile ausgewählt sind aus der Gruppe von cyanethylierten einfachen und mehrfachen Alkoholen, cyanethylierten Aminen und alpha-Aminonitrilen.

Vorteilhaft ist das erfindungsgemäße Verfahren, wenn die Hydrierung lösungsmittelfrei durchgeführt wird.

Vorteilhaft ist das erfindungsgemäße Verfahren, wenn bei Temperaturen im Bereich von 20 bis 200°C und Drücken im Bereich von 1 bis 300 bar hydriert wird.

Bei dem erfindungsgemäßen Verfahren können alle dem Fachmann bekannten Nitrile eingesetzt werden. Bevorzugt ist der Einsatz von alpha-Aminonitrilen, cyanethylierten einfachen und mehrfachen Aminen und cyanethylierten einfachen und mehrfachen Alkoholen. Besonders bevorzugt ist der Einsatz von Aminoalkyl-alpha-aminonitrilen, Alkyl-alpha-aminonitrilen, cyanethylierten Aminen, cyanethylierten Diaminen, cyanethylierten 1,2- und 1,3-Diolen, cyanethylierten Alkylalkoholen. Ganz besonders bevorzugt ist der Einsatz von Aminoacetonitril, Imidodiacetonitril, Dimethylaminoacetonitril, N,N-Dimethylaminopropylnitril, Biscyanethylglykol, 3-Methoxypropylnitril, 3-Hydroxypropylnitril, N,N-Biscyanethylethylendiamin und N,N-Biscyanoethylmethylamin. Insbesondere ganz besonders bevorzugt ist Dimethylaminoacetonitril, N,N-Dimethylaminopropylnitril, Biscyanethyldiethylendiglykol, N,N-Biscyanoethylmethylamin.

Die Temperaturen, bei denen die Hydrierung durchgeführt wird, liegen in einem Bereich von 20 bis 200°C, bevorzugt bei 60 bis 180°C, besonders bevorzugt von 80 bis 140°C, ganz besonders bevorzugt bei 90 bis 130°C.

Der bei der Hydrierung herrschende Druck liegt im Allgemeinen bei 1 bis 300 bar, bevorzugt bei 20 bis 300 bar, besonders bevorzugt bei 40 bis 240 bar, ganz besonders bevorzugt bei 80 bis 200 bar.

In einer bevorzugten Ausführungsform werden die eingesetzten Nitrile mit einer Rate der Hydrierung zugeführt, die nicht größer ist als die Rate, mit der die Nitrile mit Wasserstoff bei der Hydrierung reagiert.

Die Zuführrate ist bevorzugt so einzustellen, dass Vollumsatz erreicht wird. Dieses wird durch Temperatur, Druck, Art der Verbindung Nitrile, Menge des Katalysators, des Reaktionsmediums, Durchmischungsgüte des Reaktorinhalts, Verweilzeit etc. beeinflusst.

Das erfindungsgemäße Verfahren wird in Gegenwart eines auf ZrO₂ geträgerten Ruthenium-Katalysators durchgeführt. Der auf ZrO₂ geträgerte -Ru- Katalysator kann prinzipiell alle dem Fachmann für eine Nitrilhydrierung bekannte Formen wie Strang-, Kugel-, Tablette, Extrudate, Pulver, Split aufweisen.

Der auf ZrO₂ geträgerte -Ru- Katalysator wird vor dem Einsatz außerhalb des Reaktors oder im Reaktor durch Reduktion oxidierter Rutheniumspezies in einem Wasserstoff-enthaltendem Gasstrom bei erhöhter Temperatur aktiviert. Wenn der Katalysator außerhalb des Reaktors reduziert wird, kann danach eine Passivierung durch einen Sauerstoffenthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden.

Besonders bevorzugte ZrO₂ geträgerte-Rutheniumkatalysatoren, weisen als katalytisch aktive Masse vor der Reduktion mit Wasserstoff 0,05 bis 20 Gew.-%, bevorzugt 0,05 bis 15 Gew.-% Ruthenium bezogen auf den Gesamtkatalysator auf. Diese besonders bevorzugten auf ZrO₂ geträgerten Ru-Katalysatoren und ihre Herstellung sind in WO-A2 2015/086639 ab Seite 7, Zeile 5 bis Zeile 38 beschrieben. In WO-A2 2015/086639 wird ab Seite 5, Zeile 40 bis Seite 8, Zeile 42 auch die bevorzugten physikalischen Eigenschaften solcher auf ZrO₂ geträgerten Ru-Katalysatoren als Festbettkatalysatoren beschrieben.

Das erfindungsgemäße Verfahren kann in Gegenwart eines Lösungsmittels oder ohne Lösungsmittel durchgeführt werden. Bevorzugt ist die Durchführung ohne Lösungsmittel. Wenn jedoch in Gegenwart eines Lösungsmittels gearbeitet wird, eignen sich prinzipiell alle dem Fachmann bekannten Lösungsmittel, wobei sich die Lösungsmittel vorzugsweise gegenüber den einzusetzenden Nitrilen inert verhalten müssen.

Mögliche Lösungsmittel sind organische Lösungsmittel, beispielsweise, aromatische und aliphatische Kohlenwasserstoffe, wie Toluol, Alkohole wie Methanol, Ethanol, Propanol, Isopropanol, Butanol, Isobutanol, sekundäres Butanol und tertiäres Butanol, Amine, wie EDA bzw. Ethylamine und Ammoniak, und Ether, wie Diisopropylether, Diisobutylether, Glykoldimethylether, Diglykoldimethylether, Dioxan und Tetrahydrofuran (THF), und Amide wie N,N-Dimethylacetamid und N,N-Dimethylformamid.

Vorzugsweise ist das Lösungsmittel ein aromatischer Kohlenwasserstoff, ein Alkohol, ein Amin, oder ein Ether. Bevorzugt sind im erfindungsgemäßen Verfahren zyklische Ether und Amine. Besonders bevorzugt sind Tetrahydrofuran und Ammoniak. Ganz besonders bevorzugt ist Ammoniak.

Normalerweise wird das Nitril mit dem Lösungsmittel so vermischt, dass ein Nitrilgehalt in der Lösung von 0,5 bis 95 Gew.-% eingestellt ist. Die Konzentration der Nitrile in der Lösung, in der die Hydrierung durchgeführt wird, sollte so gewählt werden, dass eine geeignete Zuführrate bzw. Verweilzeit eingestellt werden kann. Es ist bevorzugt das Nitril mit dem Lösungsmittel so zu vermischen, dass ein Nitrilgehalt in der Lösung von 5 bis 75 Gew.-% eingestellt ist.

Die Umsetzung der Nitrile mit Wasserstoff in Gegenwart von Katalysatoren kann in üblichen für die Katalyse geeigneten Reaktionsgefäßen in einer Festbett-, Wirbelschicht-, kontinuierlich, semikontinuierlich oder diskontinuierlich durchgeführt werden. Zur Durchführung der Hydrierung eignen sich Reaktionsgefäße, in denen eine Kontaktierung der Nitrile und des Katalysators mit dem Wasserstoff unter Druck möglich ist.

Die Hydrierung an dem auf ZrO₂ geträgertem Ru-Festbettkatalysator findet bevorzugt in einem oder mehreren Rohrreaktoren aber auch Rohrbündelreaktoren statt.

Die Hydrierung der Nitrilgruppen findet unter Freisetzung von Wärme statt, die in der Regel abgeführt werden muss. Die Wärmeabfuhr kann durch eingebaute Wärmeüberträgerflächen, Kühlmäntel oder außen liegende Wärmeüberträger in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes.

Insbesondere kann der Kreislaufstrom mittels eines externen Wärmeüberträgers auf einfache und kostengünstige Weise gekühlt und somit die Reaktionswärme abgeführt werden.

Der Reaktor kann auch adiabat betrieben werden. Bei adiabatem Betrieb des Reaktors kann der Temperaturanstieg im Reaktionsgemisch durch Abkühlung der Zuläufe oder durch Zufuhr von "kaltem" organischem Lösungsmittel begrenzt werden.

Da der Reaktor selbst dann nicht gekühlt werden muss, ist eine einfache und kostengünstige Bauform möglich. Eine Alternative stellt ein gekühlter Rohrbündelreaktor dar.

Im Reaktionsaustrag gegebenenfalls vorhandene organische Lösungsmittel werden im Allgemeinen destillativ abgetrennt. Insbesondere können die erfindungsgemäßen Amine nach dem Fachmann bekannten Methoden aus dem Reaktionsprodukt isoliert werden.

Nachfolgend wird die Erfindung anhand von Beispielen verdeutlicht.

### Beispiel 1

Kontinuierliche Hydrierung von N,N-Bis(cyanethyl)methylamin (BCEMA) zu N,N-Bisaminopropylmethylamin (BAPMA) an einem Co- und Ru-Festbett-Katalysator

Durch einen bei 170 bar betriebenen vertikalen Rohrreaktor (Durchmesser: 0,5 cm, Füllhöhe 100 cm), der mit 24,6 mL eines Ruthenium-Katalysators (3 mm Stränge, beschrieben in WO-A2 2015/086639) oder 20,8 mL eines Cobalt-Katalysators (4 mm Stränge, beschrieben in EP636409) gefüllt war, wurden stündlich 4,2 bzw. 5,4 g N,N-Bis(cyanethyl)methylamin und 10,3-13,4 g flüssiger Ammoniak (Molverhältnis 20) gepumpt. Gleichzeitig leitete man 15-20 NL/h Wasserstoff durch den Reaktor.

Nach Entspannen auf Normaldruck wurde der Hydrieraustrag durch Gaschromatographie analysiert.

Die Temperatur wurde so gewählt, dass zu Beginn des Versuchs ein Umsatz von ca. 99% erreicht wurde (140°C für den Co-Katalysator, 150°C für den Ru-Kataysator).

| | Laufzeit [h] | Eintrag | Belastung [kg_{Nitril}/L_{Kat}*h] | BAPMA [GC FI-%] | Bis-BAPMA [GC FI-%] | Summe Restnitril [GC FI-%] | Umsatz [%] |
|---|---|---|---|---|---|---|---|
| Co-Kat | 1 | 1 | 0,2 | 78,8 | 9,84 | 0,8 | 99,2 |
| | 96 h | 2 | 0,2 | 73,4 | 8,4 | 6,1 | 93,9 |
| Ru-Kat | 1 | 3 | 0,2 | 92,0 | 0,9 | 0,7 | 99,3 |
| | 96 h | 4 | 0,2 | 91,3 | 1,1 | 0,8 | 99,2 |

Die Zunahme des Nitrils mit der Laufzeit bei Verwendung des Co-Katalysators zeigt, dass unter identischen Bedingungen der Ruthenium-Katalysator langsamer deaktiviert als der Cobalt-Katalysator.

### Beispiel 2

Diskontinuierliche Hydrierung von 3-{2-[2-(2-cyanoethoxy)ethoxy]ethoxy}propanenitrile (Biscyanethyldiethylendiglykol) zu 3-{2-[2-(3-aminopropoxy)ethoxy]ethoxy}propan-1-amine (TTD) an einem Co- und einem Ru-Katalysator

In einem 270 mL Autoklaven mit Strombrechern und Scheibenrührer wurden 5,0 g des entsprechenden Katalysators (ein Cobalt-Katalysator in Form von 4 mm Stränge, beschrieben in EP636409, oder ein Ruthenium-Katalysator in Form von 4 mm Strängen beschrieben wie in WO-A2 2015/086639) vorgelegt und 30 g Ammoniak aufgepresst. Der Autoklav wird auf 100° C aufgeheizt und Wasserstoff bis zu einem Gesamtdruck von 140 bar aufgepresst. Innerhalb von 15 Minuten wurde das entsprechende Nitril (6,0 g in 54 g THF) zudosiert. Die Reaktionsmischung wurde weitere 60 Minuten unter den Reaktionsbedingungen gerührt. Die mit Gaschromatographie ermittelte Zusammensetzung der nach Entspannen erhaltenen Hydrierausträge, sind in Tabelle 4 zusammengefasst.

| | TTD [GC FI-%] | Biscyanethyldiethylenglykol [GC FI-%] | |
|---|---|---|---|
| Co-Katalysator | 51,9 | 25,7 | Vergleichsbeispiel |
| 5% Ru/ZrO₂ | 72,8 | 7,03 | Erfindungsgemäßes Beispiel |

### Beispiel 3

Kontinuierliche Hydrierung von 3-{2-[2-(2-cyanoethoxy)ethoxy]ethoxy}propanenitrile (Biscyanethyldiethylendiglykol) zu 3-{2-[2-(3-aminopropoxy)ethoxy]ethoxy}propan-1-amine (TTD) an einem Ru-Festbett-Katalysator

Durch einen bei 170 bar betriebenen vertikalen Rohrreaktor (Durchmesser: 0,5 cm, Füllhöhe 100 cm), der mit 37,2 mL eines Ruthenium-Katalysators (3 mm Stränge) gefüllt war, wurden stündlich 13,5 g 3-{2-[2-(2-cyanoethoxy)ethoxy]ethoxy}propanenitrile und 33,5 g flüssiger Ammoniak (Molverhältnis 20) gepumpt. Gleichzeitig leitete man 20 NL/h Wasserstoff durch den Reaktor.

Nach Entspannen auf Normaldruck wurde der Hydrieraustrag durch Gaschromatographie analysiert.

Die Umsetzung wurde ohne signifikante Deaktivierung des Katalysators kontinuierlich über 960 Stunden betrieben.

| | Propylamin | DEG | TTD | Biscyanethyldiethylenglykol | Sonstige |
|---|---|---|---|---|---|
| 0h | 0,3 | 0,8 | 80,2 | 0,3 | 18,4 |
| 960 h | 0,5 | 1,1 | 76,8 | 0,2 | 21,4 |

### Beispiel 4

### Vergleich der Träger Al₂O₃ und ZrO₂ in semi-Batch-Fahrweise

Hydrierung von N,N-Dimethylaminopropionitril (DMAPN) zu N,N-Dimethylaminopropylamin (DMAPA)

In einem 270 mL Autoklaven mit Strombrechern und Scheibenrührer wurden 5,0 g des entsprechenden Katalysators vorgelegt und 30 g Ammoniak aufgepresst. Der Autoklav wird auf 100°C aufgeheizt und Wasserstoff bis zu einem Gesamtdruck von 140 bar aufgepresst. Innerhalb von 3 Stunden wurde das entsprechende Nitril (6,0 g in 54 g THF) zudosiert. Die Reaktionsmischung wurde weitere 60 Minuten unter den Reaktionsbedingungen gerührt. Die mit Gaschromatographie ermittelte Zusammensetzung der nach Entspannen erhaltenen Hydrierausträge, sind in Tabelle 2 und 3 zusammengefasst.

| | DMAPA [GC FI-%] | DMAPN [GC FI-%] | |
|---|---|---|---|
| 2% Ru@Al₂O₃ | 73,6 | 24,9 | Vergleichsbeispiel |
| 2% Ru@ZrO₂ | 95,7 | 0,4 | Erfindungsgemäßes Beispiel |

Hydrierung von N,N-Biscyanethylmethylamin zu N,N-Bisaminopropylmethylamin Analog der Hydrierung von Dimethylaminopropionitril

| | BAPMA [GC FI-%] | BCEMA [GC FI-%] | Mononitril [GC FI-%] | |
|---|---|---|---|---|
| 2% Ru@Al₂O₃ | 42,6 | 31,7 | 22,2 | Vergleichsbeispiel |
| 2% Ru@ZrO₂ | 73,4 | 12,7 | 9,4 | Erfindungsgemäßes Beispiel |

### Beispiel 5

### Vergleich der Träger Kohle und ZrO₂ in semi-Batch-Fahrweise

Erfindungsgemäßes Beispiel: Hydrierung von N,N-Dimethylaminoacetonitril (DMAAN) zu N,N-Dimethylaminoethylamin (DMAEA) an Ruthenium auf ZrO₂

In einem 270 mL Autoklaven mit Strombrechern und Scheibenrührer wurden 5,0 g des Katalysators (2% Ru auf ZrO₂, 3 mm Stränge) vorgelegt und 40 mL Ammoniak aufgepresst. Der Autoklav wird auf 100°C aufgeheizt und Wasserstoff bis zu einem Gesamtdruck von 140 bar aufgepresst. Innerhalb von 1,5 Stunden wurde DMAAN (6 g in 54 g THF) zudosiert. Die Reaktionsmischung wurde weitere 60 Minuten unter den Reaktionsbedingungen gerührt. Die mit Gaschromatographie ermittelte Zusammensetzung der nach Entspannen erhaltenen Hydrierausträge, sind in Tabelle 5 zusammengefasst.

Vergleichsbeispiel: Hydrierung von N,N-Dimethylaminoacetonitril (DMAAN) zu N,N-Dimethylaminoethylamin (DMAEA) an Ruthenium auf Kohle

In einem 270 mL Autoklaven mit Strombrechern und Scheibenrührer wurden 5,0 g des Katalysators (5% Ru auf Kohle) vorgelegt und 30 g Ammoniak aufgepresst. Der Autoklav wird auf 100°C aufgeheizt und Wasserstoff bis zu einem Gesamtdruck von 140 bar aufgepresst. Innerhalb von 1,5 Stunden wurde DMAAN (6 g in 54 g THF) zudosiert. Die Reaktionsmischung wurde weitere 60 Minuten unter den Reaktionsbedingungen gerührt. Die mit Gaschromatographie ermittelte Zusammensetzung der nach Entspannen erhaltenen Hydrierausträge, sind in Tabelle 5 zusammengefasst.

| | DMAEA [GC FI-%] | DMAAN [GC FI-%] | |
|---|---|---|---|
| 2% Ru@ZrO₂ | 94 | 0 | Erfindungsgemäßes Beispiel |
| 5% Ru@C | 1 | 90 | Vergleichsbeispiel |

## Patentansprüche

1. Verfahren zur Hydrierung von Nitrilen in Gegenwart von Wasserstoff und einem Ruthenium-Festbettkatalysator, der auf ZrO₂ geträgert ist.

2. Das Verfahren nach Anspruch 1, wobei der eingesetzte Ru-Festbettkatalysator 0,05 bis 20 Gew.- % Ruthenium, bezogen auf den Gesamtgewicht des Katalysators, enthält.

3. Das Verfahren nach einem der Ansprüche 1 bis 2, wobei das Nitrilhydrierverfahren kontinuierlich betrieben wird.

4. Das Verfahren nach einem der Ansprüche 1 bis 3, wobei die zu hydrierenden Nitrile Dinitrile sind.

5. Verfahren nach einem der Ansprüche 1 bis 4, wobei die zu hydrierenden Nitrile ausgewählt sind aus der Gruppe von cyanethylierten einfachen und mehrfachen Alkoholen, cyanoethylierten Aminen und alpha-Aminonitrilen sind.

6. Verfahren nach einem der Ansprüche 1 bis 5, wobei die Hydrierung lösungsmittelfrei erfolgt.

7. Verfahren nach einem der Ansprüche 1 bis 5, wobei die Hydrierung in Ammoniak erfolgt.

8. Das Verfahren nach einem der Ansprüche 1 bis 6, wobei bei Temperaturen im Bereich von 20 bis 200°C und Drücken im Bereich von 20 bis 300 bar hydriert wird.

## Claims

1. A process for hydrogenating nitriles in the presence of hydrogen and a fixed-bed ruthenium catalyst supported on ZrO₂.

2. The process according to claim 1, wherein the fixed-bed Ru catalyst used comprises 0.05 to 20 wt% of ruthenium, based on the total weight of the catalyst.

3. The process according to either of claims 1 and 2, wherein the nitrile hydrogenation process is operated continuously.

4. The process according to any of claims 1 to 3, wherein the nitriles for hydrogenation are dinitriles.

5. The process according to any of claims 1 to 4, wherein the nitriles for hydrogenation are selected from the group of cyanoethylated single and multiple alcohols, cyanoethylated amines, and alpha-aminonitriles.

6. The process according to any one of claims 1 to 5, wherein the hydrogenation takes place solventlessly.

7. The process according to any one of claims 1 to 5, wherein the hydrogenation takes place in ammonia.

8. The process according to any one of claims 1 to 6, wherein hydrogenation takes place at temperatures in the range from 20 to 200°C and pressures in the range from 20 to 300 bar.

## Revendications

1. Procédé pour l'hydrogénation de nitriles en présence d'hydrogène et d'un catalyseur à lit fixe au ruthénium, qui est supporté sur du ZrO₂.

2. Procédé selon la revendication 1, le catalyseur à lit fixe au Ru utilisé contenant 0,05 à 20 % en poids de ruthénium, par rapport au poids total du catalyseur.

3. Procédé selon l'une quelconque des revendications 1 et 2, le procédé d'hydrogénation de nitriles étant mis en œuvre en continu.

4. Procédé selon l'une quelconque des revendications 1 à 3, les nitriles devant être hydrogénés étant des dinitriles.

5. Procédé selon l'une quelconque des revendications 1 à 4, les nitriles devant être hydrogénés étant choisis dans le groupe des monoalcools et des polyols cyanoéthylés, des aminés cyanoéthylées et des alpha-aminonitriles.

6. Procédé selon l'une quelconque des revendications 1 à 5, l'hydrogénation étant réalisée sans solvant.

7. Procédé selon l'une quelconque des revendications 1 à 5, l'hydrogénation étant réalisée dans l'ammoniac.

8. Procédé selon l'une quelconque des revendications 1 à 6, dans lequel on hydrogène à des températures dans la plage de 20 à 200 °C et des pressions dans la plage de 20 à 300 bars.
